# EUROPEAN PATENT SPECIFICATION

(11) **EP 3 792 282 B1**
(45) Date of publication and mention of the grant of the patent: **02.12.2026**
(21) Application number: 20197506.7
(22) Date of filing: 09.12.2009
(51) Int. Cl.: C07K 16/24, A61K 39/395

(54) **ANTIBODY FORMULATION**
ANTIKÖRPERFORMULIERUNG
FORMULATION D'ANTICORPS

(30) Priority: 10.12.2008 EP 08170884
(43) Date of publication of application: 17.03.2021
(60) Divisional application: 26187842.5 / 4 789 682; 26189277.2 / 4 792 831
(62) Document number(s) of the earlier application(s) in accordance with Art. 76 EPC: 16162699.9 / 3 072 906; 09764858.8 / 2 376 533
(73) Proprietor: Novartis AG, 4056 Basel (CH)
(72) Inventor: MOMM, Joachim, 4002 Basel (CH); WALLNY, Hans-Joachim, 4002 Basel (CH)
(74) Representative: Carpmaels & Ransford LLP

(56) References cited:
- WO-A1-03/105894
- WO-A1-03/105894
- WO-A2-02/16436
- WO-A2-03/039485
- ALTEN RIEKE ET AL: "The human anti-IL-1Î monoclonal antibody ACZ885 is effective in joint inflammation models in mice and in a proof-of-concept study in patients with rheumatoid arthritis", ARTHRITIS RESEARCH AND THERAPY, BIOMED CENTRAL, LONDON, GB, vol. 10, no. 3, 5 June 2008 (2008-06-05), pages R67, XP021041234, ISSN: 1478-6354
- EUROPEAN MEDICINES AGENCY: "CHMP assessment report for Ilaris", INTERNET CITATION, 23 July 2009 (2009-07-23), pages 1 - 43, XP002758154, Retrieved from the Internet <URL:URL:http://www.ema.europa.eu/docs/en_GB/document_library/EPAR_-_Public_assessment_report/human/001109/WC500031679.pdf> [retrieved on 20160530]

## Description

The present invention relates to pharmaceutical formulations in which the active ingredient comprises human antibodies to human interleukin I beta (IL-1β), in particular antibody ACZ885. Antibodies, as other protein therapeutics are complex molecules and in general, large amounts of antibodies have to be used in pharmaceutical formulations due to their therapeutically effective dose in mammals, particularly humans. Liquid formulations of protein therapeutics should preserve intact the biologic activity of the protein therapeutics and protect the functional groups of the protein therapeutics from degradation during manufacturing and shelf life. Degradation pathways for proteins can involve chemical instability or physical instability. Early suggestions about how to solve the problems of instability of protein therapeutics formulations included the lyophilization of the drug product, followed by reconstitution immediately or shortly prior to administration. However, the reconstituted formulation requires being reproducible, stable and physiologically active in order to achieve a safe preparation with effective therapeutic results. Conveniently, liquid pharmaceutical formulations of protein therapeutics, i.e. antibodies should be long-term stable, contain a safe and effective amount of the pharmaceutical compound. A long appreciated problem with liquid formulations of protein therapeutics is that of aggregation, where protein molecules physically stick together, for example, resulting in the formation of opaque insoluble matter or precipitation, which may show undesired immunological reactions. Additionally, a major problem caused by the aggregate formation is that during the administration the formulation may block syringes or pumps and rendering it unsafe to patients.

WO 02/16436 A (NOVARTIS AG) discloses pharmaceutical compositions comprising a human antibody to interleukin I beta, ACZ885. The pharmaceutical composition is preferably provided in lyophilized form, and is dissolved in a suitable aqueous carrier, for example sterile water for injection or sterile buffered physiological saline. Thus, there is a need for formulations comprising protein therapeutics, in particular antibodies that are long-term stable, free of aggregation at high antibody concentrations. The present invention addresses the above-identified need by providing a novel formulation comprising an antibody, free of protein aggregates, stable and having sufficiently low viscosity and which is therefore suitable for administration to mammalians, particularly human subjects. Interleukin-1β (IL-1beta or IL-1β or Interleukin-1β have the same meaning herein) is a potent immuno-modulator which mediates a wide range of immune and inflammatory responses. Inappropriate or excessive production of IL-1β is associated with the pathology of various diseases and disorders, such as septicemia, septic or endotoxic shock, allergies, asthma, bone loss, ischemia, stroke, rheumatoid arthritis and other inflammatory disorders. Antibodies to IL-1β have been proposed for use in the treatment of IL-1 mediated diseases and disorders; see for instance, WO 95/01997 and the discussion in the introduction thereof and WO 02/16436. Alten et al. (2008) Arth Res Ther 10:R67 reports that ACZ885 is effective in joint inflammation models in mice and in a proof-of-concept study in patients with rheumatoid arthritis. ACZ885 is also the topic of the CHMP assessment report for Ilaris (canakinumab) published by the European Medicines Agency in July 2009.

The antibody to IL-1β for the formulations of the present invention is the ACZ885 antibody as hereinafter described in Seq. Id. No. 1 and Seq. Id. No. 2, or functional fragments thereof retaining affinity for the antigen, such as F(ab)2, Fab, scFv, VH domains, CDR's.

WO03/039485 discloses a stable liquid pharmaceutical formulation comprising a high concentration, e.g. 50 mg/ml or more, of antibody in about 20-60 mM succinate buffer or 30-70 mM histidine buffer, having pH from about pH 5.5 to about pH 6.5, about 0.01-0.1 % polysorbate, and a tonicity modifier that contributes to the isotonicity of the formulation.

WO03/105894 discloses liquid formulations of SYNAGIS^{®} which are substantially free of surfactant, inorganic salts, and/or other common excipients. The formulation used in a clinical study had 100 mg/ml of antibody with 25 mM histidine and 1.6 mM glycine at pH 6.

It is an object of the present invention to provide an antibody formulation which is stable upon storage and delivery. According to the present invention a stable formulation is a formulation wherein the antibody therein essentially retains its physical and chemical stability and integrity upon storage. For example, the extent of product related substances and impurities following lyophilization and storage or storage in the case of liquid formulation is about 2-5%, preferably 2-3%. The stability of the antibody formulation may be measured using biological activity assays and wherein the biological activity upon storage is of about 80-125% of the original activity. The biological activity of the antibody in the formulation of the invention upon storage is measured in a reporter gene assay, using the genetically modified cell line as described in the examples section hereinafter.

In general, it is preferred to use small volumes of pharmaceutical formulation for subcutaneous injection (usually 1.0 mL-1.2 mL at a maximum). In the case of formulations comprising antibodies, e.g. high dose antibody therapies, the subcutaneous administration requires high concentration antibody formulations (e.g., 50 mg/ml-150 mg/ml or more). Because of the required high antibody concentrations, the formulations comprising antibodies pose challenges relating to the physical and chemical stability of the antibody, formation of aggregates and difficulty with manufacture, storage, and delivery of the antibody formulation.

Increased viscosity of protein formulations has negative implications from processing, e.g. processability of the liquid through drug delivery to the patient, e.g. at high viscosity, the liquid formulation do not longer pass through the gauge of a needle without difficulty, causing discomfort to the patient; injection duration; utilizability of auto-injector. Additionally relatively high concentration antibody formulations with suitably low viscosities are desired as a prerequisite for easy manufacturing, storage, and administration. The term "viscosity" as used herein, may be "kinematic viscosity" or "absolute viscosity." Commonly, kinematic viscosity is expressed in centistokes (cSt). The SI unit of kinematic viscosity is mm²/s, which is 1 cSt. Absolute viscosity is expressed in units of centipoise (cP). The SI unit of absolute viscosity is the millipascal-second (mPa-s), where 1 cP=1 mPa-s.

Therefore the present invention provides: (i) a liquid pharmaceutical formulation comprising 10 to 150 mg/mL ACZ885, 270 mM sucrose, 20 to 30 mM histidine, and 0.06% polysorbate 80, wherein the pH of the formulation is 6.5; and (ii) a lyophilisate which in reconstituted form yields a reconstituted formulation comprising 10-150 mg/mL ACZ885, 270 mM sucrose, 20 to 30 mM histidine, and 0.06% polysorbate 80, wherein the pH of the formulation is 6.5.

A liquid pharmaceutical antibody formulation should exhibit a variety of pre-defined characteristics. One of the major concerns in liquid drug products is stability, as proteins tend to form soluble and insoluble aggregates during manufacturing and storage. In addition, various chemical reactions can occur in solution (deamidation, oxidation, clipping, isomerization etc.) leading to an increase in degradation product levels and/or loss of bioactivity. Preferably, a liquid antibody formulation should exhibit a shelf life of more than 18 months Most preferred a liquid ACZ885 formulation should exhibit a shelf life of more than 24 months. The shelf life and activity of an IL-1β antibody is defined in the bioactivity assay in the examples section, whereby the activity should remain between 80% and 125% of the original activity.

An antibody formulation, in particular ACZ885 antibody formulation should exhibit a shelf life of about 36 to 60 months at 2-8 °C. Preferably, the ACZ885 lyophilizate formulation should exhibit a shelf life of about preferably up to 60 months at 2-8 °C.

The main factors determining shelf life usually are formation of by- and degradation products and loss of bioactivity. The formulation of the current invention achieves these desired stability levels.

Apart from sufficient physical and chemical stability the formulation should be of acceptable pH value and osmolality (250 to 500 mOsm/kg) for subcutaneous application. However it was reported in literature that preparations with high osmolality (up to 1100 mOsm/kg) could be administered subcutaneously without significantly increased pain perception or burning duration after injection. It is also known that high concentration of antibodies would increase the viscosity of the formulation and also the aggregation. Suitable pharmaceutical formulation according to the invention have a viscosity of about less than 16 mPas, preferably 3 to 16 mPas, and preferred 3-10 mPas.

In accordance with the present invention it has now surprisingly been found that particularly stable antibody formulations are obtainable that have advantageous properties in preserving antibody activity of long period of storage, avoiding aggregation and having a suitable viscosity despite high antibody concentrations. A reconstituted formulation is a formulation which has been prepared from a lyophilizate, such that the antibody of the formulation is dispersed in the reconstituted formulation

Best results were obtained using histidine.

In the formulation, the buffer system is histidine; and histidine is used at a concentration from 20 to 30 mM.

The formulation of the invention further comprises a stabilizer, namely 270 mM of sucrose.

The use of a surfactant can reduce aggregation of the reconstituted protein and/or reduce the formation of particulates in the reconstituted formulation. The amount of surfactant added is such that it reduces aggregation of the reconstituted protein and minimizes the formation of particulates after reconstitution. The amount of surfactant is 0.06% polysorbate 80.

Other pharmaceutically acceptable carriers, excipients or stabilizers such as those described in Remington's Science and Practice of Pharmacy 21st edition, (2005) or Art, Science and Technology of Pharmaceutical Compounding, 3rd edition (2008) may be included in the formulation of the invention provided that they do not adversely affect the desired characteristics of the formulation. Acceptable carriers, excipients or stabilizers are nontoxic to recipients at the dosages and concentrations employed and include additional buffering agents; preservatives; co-solvents; antioxidants including ascorbic acid and methionine; chelating agents such as EDTA; metal complexes (e.g. Zn-protein complexes); biodegradable polymers such as polyesters; and/or salt-forming counterions such as sodium.

As reconstitution media several diluted organic acids or water, i.e. sterile water, bacteriostatic water for injection (BWFI) may be used. The reconstitution medium may be selected from water, i.e. sterile water, bacteriostatic water for injection (BWFI) or the group consisting of acetic acid, propionic acid, succinic acid, sodium chloride, magnesium chloride, acidic solution of sodium chloride, acidic solution of magnesium chloride and acidic solution of arginine, in an amount from about 50 to about 100 mM.

A preferred reconstituted formulation of the invention provides a lyophilisate which in reconstituted form yields a reconstituted formulation comprising ACZ885 at concentration: 10-150 mg/ml, 270mM sucrose, 30mM histidine and 0.06% polysorbate 80, wherein the pH of the formulation is 6.5.

Reconstituted formulations may be used in a method for treating a mammal, particularly human subject comprising administering a therapeutically effective amount of a reconstituted formulation disclosed herein to a mammal, particularly human subject wherein the mammal, particularly human subject has a disorder requiring treatment with ACZ885. For example, the formulation may be administered subcutaneously.

Formulations of the invention are useful for the prophylaxis and treatment of IL-1 mediated diseases or medical conditions, e.g. inflammatory conditions, allergies and allergic conditions, hypersensitivity reactions, autoimmune diseases, severe infections, and organ or tissue transplant rejection.

The formulation is useful for the treatment of IL-1 mediated diseases or medical conditions. For example, formulations of the invention may be use for the treatment of recipients of heart, lung, combined heart-lung, liver, kidney, pancreatic, skin or corneal transplants, including allograft rejection or xenograft rejection, and for the prevention of graft-versus-host disease, such as following bone marrow transplant, and organ transplant associated arteriosclerosis.

Formulations of the invention are particularly useful for the treatment, prevention, or amelioration of autoimmune disease and of inflammatory conditions, in particular inflammatory conditions with an aetiology including an autoimmune component such as arthritis (for example rheumatoid arthritis, arthritis chronica progrediente and arthritis deformans) and rheumatic diseases, including inflammatory conditions and rheumatic diseases involving bone loss, inflammatory pain, hypersensitivity (including both airways hypersensitivity and dermal hypersensitivity) and allergies. Specific auto-immune diseases for which formulations of the invention may be employed include autoimmune haematological disorders (including e.g. hemolytic anaemia, aplastic anaemia, pure red cell anaemia and idiopathic thrombocytopenia), systemic lupus erythematosus, polychondritis, sclerodoma, Wegener granulomatosis, dermatomyositis, chronic active hepatitis, myasthenia gravis, psoriasis, Steven-Johnson syndrome, idiopathic sprue, autoimmune inflammatory bowel disease (including e.g. ulcerative colitis, Crohn's disease and Irritable Bowel Syndrome), endocrine ophthalmopathy, Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy).

Formulations of the invention are also useful for the treatment, prevention, or amelioration of asthma, bronchitis, pneumoconiosis, pulmonary emphysema, and other obstructive or inflammatory diseases of the airways

Formulations of the invention are useful for treating undesirable acute and hyperacute inflammatory reactions which are mediated by IL-1 or involve IL-1 production, especially IL-1β, or the promotion of TNF release by IL-1, e.g. acute infections, for example septic shock (e.g., endotoxic shock and adult respiratory distress syndrome), meningitis, pneumonia; and severe burns; and for the treatment of cachexia or wasting syndrome associated with morbid TNF release, consequent to infection, cancer, or organ dysfunction, especially AIDS -related cachexia, e.g., associated with or consequential to HIV infection.

Formulations of the invention are particularly useful for treating diseases of bone metabolism including osteoarthritis, osteoporosis and other inflammatory arthritides, and bone loss in general, including age-related bone loss, and in particular periodontal disease.

Formulations of the invention are useful in the prevention and treatment of Auto-Inflammatory Syndromes in patients such as in mammals, particularly humans. Auto-Inflammatory Syndromes according to the inventions are e.g., but not limited to, a group of inherited disorders characterized by recurrent episodes of inflammation, that in contrast to the auto-immune diseases lack high-titer autoantibodies or antigen specific T cells. Furthermore, Auto-inflammatory Syndromes according to the inventions show increased IL-1 beta secretion (loss of negative regulatory role of pyrin which seems mutated in said diseases), NFkB activation and impaired leukocyte apoptosis). Auto-inflammatory Syndromes according to the inventions are Muckle-Wells syndromes (MWS), LADA (Latent Autoimmune Diabetes in Adults), familial cold autoinflammmatory syndrome (FCAS), Cryopyrin-associated periodic syndromes (CAPS), neonatal-onset mutlisystem inflammatory syndrome (NOMID), chronic infantile neurological, cutaneous, articular (CINCA) syndrome, familial Mediterranean fever (FMF) and/or certain form of juvenile arthritis such as systemic onset juvenile idiopathic arthritis (SJIA), certain form of juvenile rheumatoid arthritis such as systemic onset juvenile idiopathic rheumatoid arthritis and/or certain form of adult rheumatoid arthritis.

Preferably the formulations of the invention are useful in the prevention and treatment of Juvenile rheumatoid arthritis and adult rheumatoid arthritis and/or Muckle Wells Syndrome. The formulations of the invention may also be useful in the treatment of type 2 diabetes, where clinical and preclinical studies show improved islet function by IL-1 blockade. Formulations of the invention are also useful in the treatment of various diabetes related pathologies such as retinopathy, wound healing, vascular diseases, (incl. arterial restenosis after stenting or angioplasty), renal dysfunction, chronic kidney disease and metabolic syndrome and obesity. The formulations of the invention may also be useful in the treatment of migraine, synovitis, gout, pseudogout / gouty arthritis or chondrocalcinosis, chronic obstructive pulmonary disease (COPD), ventilation induced lung damage, various pain conditions, such as morphine resistant pain, neuropathic pain, pre-term birth pain, discogenic pain, inflammatory pain, headache, or migraine. IL-1 beta is involved in pain perception and amplifies neurogenic signals. Furthermore formulations of the invention of the invention are useful in the treatment of atherosclerosis, acute renal colic, biliary colic and pain related to these disorders.

The formulations of the invention may be useful in the treatment of Periodic Fever Syndromes: Familial Mediterranean Fever (FMF), Tumor Necrosis Factor Receptor Associated Periodic Syndrome (TRAPS), Hyperimmunoglobulin D syndrome (HIDS), also called Mevalonate Kinase Associated Periodic Fever Syndrome, Familial Cold auto inflammatory syndrome and Periodic fever, Aphthous-stomatitis, Pharyngitis, Adenitis (PFAPA) Syndrome, where IL-1 beta is a dominant cytokine. Other diseases wherein IL-1 beta is a dominant cytokine and that can be treated with formulations of the invention according to the invention comprise Anti-synthetase syndrome, Macrophage activation syndrome MAS, Beh 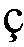 et Disease, Blau's syndrome, PAPA syndrome, Schnitzler's syndrome, Sweet's syndrome. IL-1beta ligand - receptor blocking and IL-1beta compounds of the invention may also be used to treat Vasculitides; Giant-cell arteritis (GCA), Henoch-Schoenlein purpura, Primary systemic vasculitis, Kawasaki disease (mucocutaneous lymph node syndrome), Takayasu arteritis, Polyarteritis nodosa, Essential cryoglobulinemic vasculitis, microscopic polyangiitis (MPA) and Churg-Strauss syndrome (CSS), urticarial vasculitis. Furthermore formulations of the invention are useful in the treatment of autoimmune diseases like sarcoidosis, pemphygus, ankylosing spondylitis, Alzheimer disease, amyloidosis, secondary amyloidosis and adult onset Still disease (AOSD).

Formulations of the invention may be used to treat HLA-B27 associated diseases such as but not limited to psoriatica, spondylitis ankylosans, Morbus Reiter and enteropathic arthritis. IL-1beta compounds according to the invention may be used to treat rheumatic fever, polymyalgia rheumatica and giant cell arteritis. Finally formulations of the invention may be used to treat infections, in particular bacterial infections and viral infections, more in particular bacterial infections associated with arthritic symptoms or observations, such as but not limited to hematogenic osteomyelitis, infectious arthritis, tuberculotic arthritis.

For the above indications, the appropriate dosage will vary depending upon, for example, the particular antibody to IL-1β to be employed, the host, the mode of administration and the nature and severity of the condition being treated. The frequency of dosing for prophylactic uses will normally be in the range from about once per week up to about once every 3 months, more usually in the range from about once every 2 weeks up to about once every 10 weeks, e.g. once every 4 to 8 weeks.

The formulation of the invention is suitably administered to the patient at one time or over a series of treatments and may be administered to the patient at any time from diagnosis onwards; it may be administered as the sole treatment or in conjunction with other drugs or therapies useful in treating the conditions as described herein before.

A prophylactic treatment typically comprises administering the formulation of the invention once per month to once every 2 to 3 months, or less frequently.

The formulation of the invention comprising ACZ885 is administered preferably by intravenous route, but also by subcutaneous or intramuscular injection route. For such purposes, the formulation may be injected using a syringe. For example, the formulation comprising ACZ885 is administered using autoinjector, normal syringe that may be prefilled, optionally in a sterile package, optionally syringes with safety devices. Micro-needle and coated patches with reservoirs are also envisaged as suitable administration devices.

The formulation of the invention may be administered to a mammal, preferably a human in need of treatment with ACZ885, in accord with known methods, such as intravenous administration as a bolus or by continuous infusion over a period of time, by intramuscular, intraperitoneal, intracerebrospinal, subcutaneous, intra-articular, intrasynovial, intrathecal, oral, topical, or inhalation routes.

The formulations to be used for in vivo administration must be sterile. This is readily accomplished by filtration through sterile filtration membranes, prior to, or following, lyophilization and reconstitution. Alternatively, sterility of the entire mixture may be accomplished by autoclaving the ingredients, except for antibody, at about 120°C for about 30 minutes, for example.

The formulation of the invention comprising ACZ885 is preferably administered by subcutaneous injection in treatments of rheumatoid arthritis in adults (RA), juvenile RA (SJIA, pJIA), chronic obstructive pulmonary disease (COPD), CAPS, Muckle-Wells syndrome (MWS), Osteoarthritis (OA) and potentially type 2 diabetes and gout.

An isotonic liquid formulation can be obtained by dilution of a reconstituted formulation with an infusion solution.

The term treatment refers to both therapeutic treatment and prophylactic or preventative measures.

The term mammal for purposes of treatment refers to any animal classified as a mammal, including humans, domestic and farm animals, and zoo, sports, or pet animals, such as dogs, horses, cats, cows, etc. Preferably, the mammal is human.

A disorder is any condition that would benefit from treatment with an antibody to IL-1β. This includes chronic and acute disorders or diseases including those pathological conditions which predispose the mammal to the disorder in question. Non-limiting examples of disorders to be treated herein include the above-mentioned diseases and disorders.

A therapeutically effective amount is at least the minimum concentration required to effect a measurable improvement or prevention of a particular disorder.

Figure Legends:
Figure 1 shows an overview of the results obtained by RP-HPLC (top) and SEC (bottom) for ACZ885 formulation after 4 week storage at 40°C.

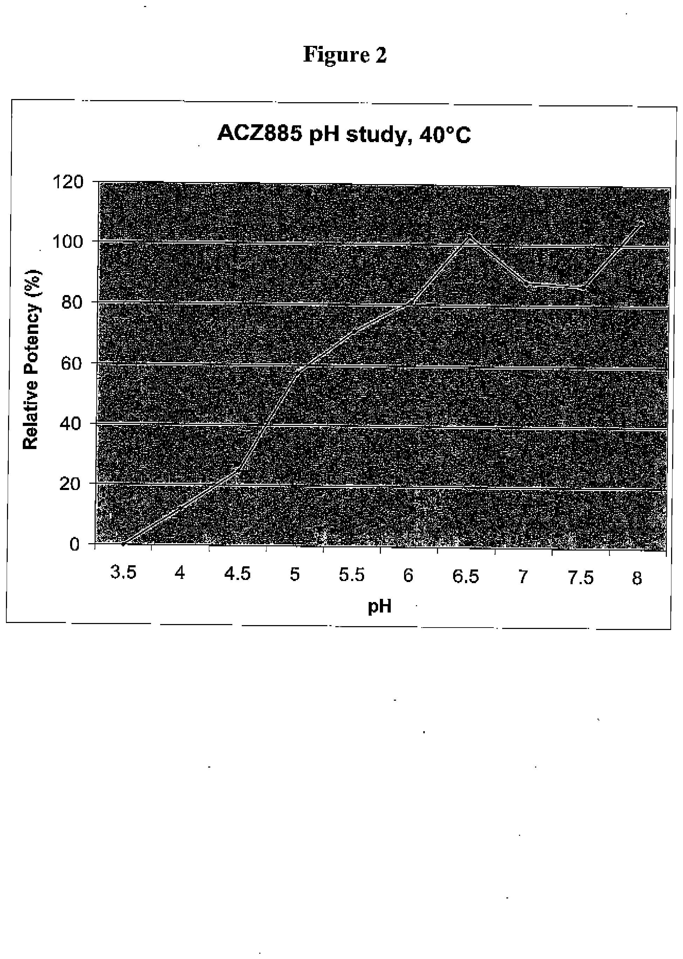

Figure 2 shows an overview of the results obtained for the relative potency for ACZ885 formulation after 4 week storage at 40°C

### EXAMPLES

### Preparation of a Liquid Formulation and its Lyophilisate

An ACZ885 formulation is developed that allows for both intravenous administration after reconstitution and subsequent dilution and subcutaneous administration after reconstitution. Four different buffer systems (citrate, histidine, sodium succinate, and sodium/potassium phosphate buffer systems, concentration 40 mM each) were selected to test their suitability for ACZ885 formulations.

Shaking and freeze thaw cycles are used as stress tests to rank order suitability buffer systems with regard to protein aggregation.

Protein aggregation could most efficiently be avoided using histidine or citrate buffer at a pH range of 5.0 - 7, as shown in the table 1.

**Table 1 Analytical results of buffer system screening**

| **Buffer salt** | **pKa values** | **pH (planned)** | **pH (measured)** | **Sum of Aggregates by SEC [%]** |
|---|---|---|---|---|
| Histidine | pKₐ = 6.1, imidazole group | 6.0 | 5.8 | 0.37 |
| | | 6.5 | 6.2 | 0.21 |
| | | 7.0 | 6.6 | 0.38 |
| Succinate | pKₐ₁ = 4.19, pKₐ₂ = 5.57 | 5.0 | 5.2 | 0.39 |
| | | 5.5 | 5.6 | 0.39 |
| | | 6.0 | 6.0 | 0.42 |
| Phosphate | pKₐ₂ = 7.21 | 6.0 | 6.2 | 0.47 |
| | | 6.5 | 6.7 | 0.74 |
| | | 7.0 | 7.0 | 3.94 |
| Citrate | pKₐ₂ = 4.76, pKₐ₃ = 6.40 | 5.0 | 5.3 | 0.38 |
| | | 5.5 | 5.7 | 0.40 |
| | | 6.0 | 6.2 | 0.39 |

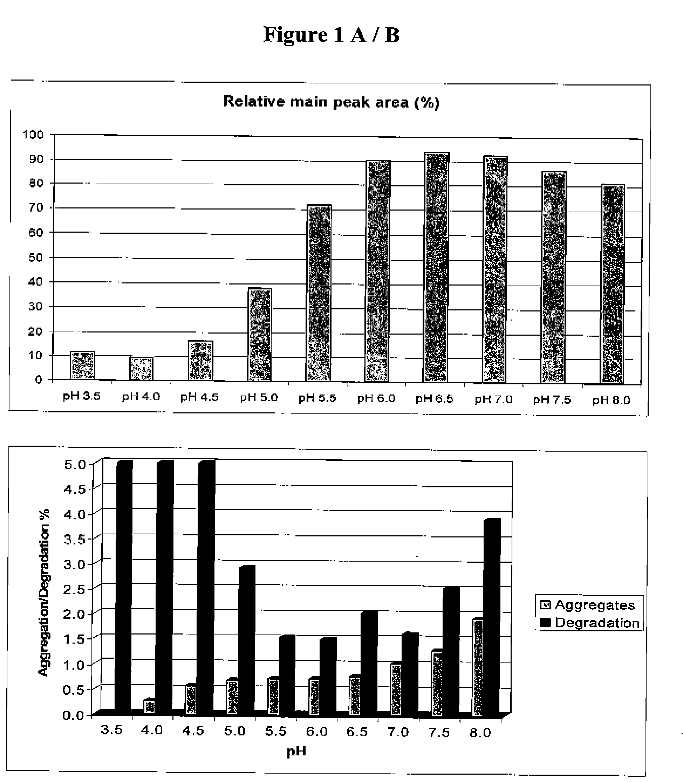

A pH range of 3.5 to 8.0 with increasing steps of 0.5 units was investigated. After 4 week storage at 40°C different pH optima of 6.2 to 6.8 were concluded based on the results from different analytical techniques applied, as shown in Figure 1.

**Table 2 Analytical results for lyophilisation**

| **Code** | **Buffer (20 mM)** | **Sucrose added** | **Viscosity [mPas]** | **Osmolarity [mOsm]** | **Sum of Aggregates by SEC [%]** |
|---|---|---|---|---|---|
| F1 | Histidine pH 6.2 | **-**- | 14.8 | 382 | 5.4 |
| **F2** | **Histidine pH 6.2** | **90 mM** | **11.0** | **392** | **0.6** |
| F3 | Citrate pH 6.0 | **-**- | 13.2 | 386 | 3.9 |
| **F4** | **Citrate pH 6.0** | **90 mM** | **11.0** | **445** | **0.6** |

The results for histidine and the citrate buffered samples were similar. Citrate buffer can be critical in subcutaneous formulations due to increased pain perception after application; therefore histidine may be preferred over citrate for subcutaneous applications.

After selection of suitable buffer systems, the impact of stablilizers on protein aggregation was investigated. Formulations containing sucrose, glycine, mannitol, sorbitol or trehalose were analyzed after 6 and 16 weeks storage at 5°C and 40°C. A yellow coloration was observed for sucrose containing formulations stored at 40°C, probably due to Maillard reaction between amino groups of protein or histidine and the reducing sugar. Sucrose or mannitol containing formulation were selected for lyophilizate and reconstituted formulation. The formulations containing mannitol were selected for the liquid formulation.

Further studies were performed to assess the influence of surfactant concentration on the physico-chemical stability of the formulation of the invention. The particulate matter data below showed the highest values for the surfactant free formulation, indicating that Tween is beneficial for physical stability of the samples. At a concentration of 0.10% Tween, particulate matter data tend to be higher compared to the lower levels.

**Table 3 Analytical results for Tween: Subvisible Particles by Light Obscuration (particulate matter) after 10 months**

| **Concentration Tween** | **5°C** | | | **25°C** | | |
|---|---|---|---|---|---|---|
| | **Particles / mL** | **Particles / mL** | **Particles / mL** | **Particles / mL** | **Particles / mL** | **Particles / mL** |
| | **> 1.0 µm** | **> 1.0 µm** | **> 10.9 µm** | **> 25.7 µm** | **> 10.9 µm** | **> 25.7 µm** |
| no Tween | 6353 | 11644 | 14 | 0 | 3 | 0 |
| 0.01% Tween 80 | 862 | 2044 | 0 | 0 | 2 | 0 |
| 0.04% Tween 80 | 1217 | 2077 | 2 | 0 | 0 | 0 |
| 0.10% Tween 80 | 2180 | 2424 | 0 | 0 | 3 | 0 |
| 0.01% Tween 20 | 1349 | 2105 | 0 | 0 | 0 | 0 |
| 0.04% Tween 20 | 1410 | 1077 | 1 | 0 | 2 | 0 |
| 0.10% Tween 20 | 1071 | 2382 | 2 | 0 | 2 | 0 |

Samples of liquid formulation containing 150 mg/mL ACZ885, 20 mM Histidine, 270 mM Mannitol, 0.04% (m/v) Tween 80, having a pH 6.5 were stored at 5°C, 25°C and 40°C for up to 24 months. At 5°C, no major amounts of both soluble and insoluble aggregates could be detected. Bioactivity, determined using the reporter gene assay as hereinafter described, was within 70 - 125%. These data (see Tables 4 to 6) showed that the formulation tested was stable upon storage for 24 months.

**Table 4 Analytical Data of Screen, 5°C**

| **Storage periods** | **Start** | | **2 months** | | **4 months** | | **10 months** | | **16.5 months** | | **24 months** | |
|---|---|---|---|---|---|---|---|---|---|---|---|---|
| Assay by SEC [mg/mL] | 152.2 | | 155.0 | | 151.9 | | 153.0 | | 153.0 | | 151.9 | |
| Reporter Gene Assay [%] | 121 | | n. d. | | 97 | | 99 | | 110 | | 99 (n=4) | |
| Appearance of the solution: color | Colorless | | Colorless | | Slightly brownish (B8-B9) | | Slightly brownish (B8-B9) | | Slightly brownish (B8) | | Brownish (B5-B6) | |
| | | | | | | | | | | | No particles visible | |
| Turbidity | n. d. | | n. d. | | n. d. n. d. | | | | strongly opalescent (23 NTU) | | opalescent (17 NTU) | |
| LLS [Da] | 155'750 | | 151'800 | | 147'400 | | 142'300 | | 147'800 | | 155'700 | |
| pH value | 6.7 | | 6.7 | | 6.6 | | 6.6 | | 6.6 | | 6.6 | |
| By- and de-gradation products by SEC [%] | AP1: | 0.3 | AP1: | 0.4 | AP1: | 0.4 | AP1: | 0.6 | AP1: | 0.6 | AP1: | 0.8 |
| | - | | - | | DPx: | 1.2 | DPx: not resolved | | DPx: not resolved | | DPx: | 0.2 |
| | - | | - | | - | | - | | - | | DP3: | 0.2 |
| Impurities by SDS-PAGE [%] | 0.8 | | 0.8 | | 0.8 | | 0.8 | | 0.9 | | 1.1 | |
| | n.d. | | n.d. | | n.d. | | n.d. | | H1: | 0.7 | H1: | 0.7 |
| Impurities by Bioanalyzer [%] | | | | | | | | | M1: | 3.1 | M1: | 2.1 |
| | | | | | | | | | M2: | 0.3 | M2: | 0.0 |
| | | | | | | | | | M3: | 0.7 | M3: | 0.6 |
| | | | | | | | | | L1: | 0.0 | L1: | 0.0 |
| | | | | | | | | | L2: | 0.3 | L2: | 0.3 |
| | | | | | | | | | sum: | 5.2 | sum: | 3.8 |
| CEX [%] | n.d. | | n.d. | | n.d. | | n.d. | | 0K: | 48.7 | 0K: | 43.4 |
| | | | | | | | | | 1K: | 19.6 | 1K: | 19.1 |
| | | | | | | | | | 2K: | 15.8 | 2K: | 10.5 |
| | | | | | | | | | Sum Other: 15.9 | | Sum Other: 27.0 | |

| | | | | | | | | | | | | |
|---|---|---|---|---|---|---|---|---|---|---|---|---|
| APx to DP3: from higher to lower molecular weight; AP1: Dimer, DPx: P100, DP3: P50 | | | | | | | | | | | | |

**Table 5 Analytical Data of Screen, 25°C**

| **Storage periods** | **Start** | | **2 months** | | **4 months** | | **10 months** | | **16.5 months** | | **24 months** | |
|---|---|---|---|---|---|---|---|---|---|---|---|---|
| Assay by SEC [mg/mL] | 152.2 | | 146.7 | | 145.8 | | 149.8 | | 141.8 | | 134.2 | |
| Reporter Gene Assay [%] | 121 | | n.d. | | 100 | | 85 | | 74 | | 70 (n=4) | |
| Appearance of the solution: color | Colorless | | Colorless | | Slightly brownish (B8-B9) | | Slightly brownish (B8-B9) | | Slightly brownish (B6) | | Brownish (B5-B6) | |
| | | | | | | | | | | | No particles visible | |
| Turbidity | n.d. | | n.d. | | n.d. | | n.d. | | strongly opalescent (22 NTU) | | strongly opalescent (24 NTU) | |
| LLS [Da] | 155'750 | | 150'250 | | 152'250 | | 148'700 | | 172'250 | | 189'800 | |
| pH value | 6.7 | | 6.7 | | 6.6 | | 6.6 | | 6.6 | | 6.6 | |
| By- and de-gradation products by SEC [%] | - | | - | | - | | - | | - | | APx: | 0.4 |
| | - | | - | | - | | - | | - | | AP2: | 0.2 |
| | AP1: | 0.3 | AP1: | 0.7 | AP1: | 0.9 | AP1: | 1.8 | AP1: | 2.6 | AP1: | 3.7 |
| | - | | - | | - | | AP: | 0.2 | AP: | 0.2 | AP: | 0.5 |
| | - | | - | | DPx: | 1.4 | DPx: | 3.7 | DPx: | 1.7 | DPx: | 2.6 |
| | - | | DP3: | 0.1 | DP3: | 0.2 | DP3: | 0.6 | DP3: | 1.1 | DP3: | 1.5 |
| Impurities by SDS-PAGE [%] | 0.8 | | 1.5 | | 1.7 | | 3.6 | | 4.2 | | 6.7 | |
| Impurities by Bioanalyzer [%] | n.d. | | n.d. | | n.d. | | n.d. | | H1: | 1.7 | H1: | 2.1 |
| | | | | | | | | | H2: | 0.0 | H2: | 0.4 |
| | | | | | | | | | H3: | 0.0 | H3: | 0.3 |
| | | | | | | | | | M1: | 4.1 | M1: | 3.7 |
| | | | | | | | | | M2: | 1.0 | M2: | 1.1 |
| | | | | | | | | | M3: | 0.7 | M3: | 0.7 |
| | | | | | | | | | M4: | 0.3 | M4: | 0.0 |
| | | | | | | | | | L1: | 0.4 | L1: | 0.6 |
| | | | | | | | | | L2: | 0.8 | L2: | 0.7 |
| | | | | | | | | | sum: | 8.9 | sum: | 9.7 |
| CEX [%] | n.d. | | n.d. | | n.d. | | n.d. | | 0K: | 36.2 | 0K: | 12.6 |
| | | | | | | | | | 1K: | 16.6 | 1K: | 6.7 |
| | | | | | | | | | 2K: | 11.0 | 2K: | 3.3 |
| | | | | | | | | | Sum Other: 36.2 | | Sum Other: 77.4 | |

| | | | | | | | | | | | | |
|---|---|---|---|---|---|---|---|---|---|---|---|---|
| APx to DP3: from higher to lower molecular weight; AP1: Dimer, DPx: P100, DP3: P50 | | | | | | | | | | | | |

**Table 6 Analytical Data of Screen, 40°C**

| **Storage periods** | **Start** | | **2 months** | | **4 months** | | **10 months** | | **16.5 months** | |
|---|---|---|---|---|---|---|---|---|---|---|
| Assay by SEC [mg/mL] | 152.2 | | 142.9 | | 128.1 | | 114.1 | | 94.5 | |
| Reporter Gene Assay [%] | 121 | | 78 | | 53 | | n.d. | | n.d. | |
| Appearance of the solution: color | Colorless | | Slightly brownish (B7) | | Slightly brownish (B6-B7) | | Yellow (G4-G5) | | Brownish (B4) | |
| Turbidity | n.d. | | n.d. | | n.d. | | n.d. | | out of range (58 NTU) | |
| LLS [Da] | 155'750 | | 162'950 | | 192'500 | | 259'650 | | 403'350 | |
| pH value | 6.7 | | 6.6 | | 6.6 | | 6.6 | | 6.6 | |
| By- and de-gradation products by SEC [%] | - | | APx: | 0.4 | APx: | 1.9 | APx: | 4.4 | APx: | 6.1 |
| | - | | AP2: | 0.1 | AP2: | 0.3 | AP2: | 1.4 | AP2: | 2.1 |
| | AP1: | 0.3 | AP1: | 2.8 | AP1: | 5.0 | AP1: | 10.2 | AP1: | 15.0 |
| | - | | AP: | 0.2 | AP: | 0.8 | AP: | 2.2 | AP: | 3.3 |
| | - | | - | | DPx: | 5.5 | DPx: | 11.7 | DPx: | 12.2 |
| | - | | DP3: | 0.9 | DP3: | 1.9 | DP3: | 4.4 | DP3: | 8.6 |
| Impurities by SDS-PAGE [%] | 0.8 | | 5.8 | | 6.7 | | 12.5 | | 13.3 | |
| CEX [%] | n.d. | | n.d. | | n.d. | | n.d. | | see chromatogram | |

| | | | | | | | | | | |
|---|---|---|---|---|---|---|---|---|---|---|
| APx to DP3: from higher to lower molecular weight; AP1: Dimer, DPx: P100, DP3: P50 | | | | | | | | | | |

### Reconstitution of the Lyophilized Formulation

Liquid formulations for ACZ885 according to the invention are suitable for lyophilization. Lyophilisation can be done under normal conditions well known in the art of pharmacy. A bulking agent may be included add weight and visibility to the lyophilisate, such as glycine. After the antibody, buffer agent, in an amount from 10 to 40 mM), stabilizer and surfactant are mixed together, the formulation is lyophilized.

**Table 7 Formulations for technical stability (before lyophilization)**

| **Code** | **Sucrose [mM]** | **Histidine pH 6.0 - 6.2 [mM]** | **Tween 80 [%]** |
|---|---|---|---|
| F1 | 60 | 10 | 0.02 |
| F3 | 90 | 10 | 0.02 |

Reconstitution generally takes place at a temperature 15-25°C to ensure complete hydration.

The lyophilizate is reconstituted with sterile water.

The target concentration after reconstitution is 150 mg/ml, each formulation being lyophilised and reconstituted with water.

### Stability

Different formulations are stored for three months at 2 - 8 °C, 25 °C and 40 °C.

The aggregation following lyophilization and storage is used as an indicator of protein stability.

### Reporter Gene Assay

The biological activity of ACZ885 was measured in a reporter gene assay, using the genetically modified cell line. This cell line was derived from the human embryonic kidney cell, and was stably transfected with a reporter construct in which the promoter NF-kappa b (an IL-1β-responsive promoter) was fused upstream of the luciferase gene. Transfection was done by co introduction of a neomycin resistance gene. In this cell line, exposure to IL-1β stimulated the expression of luciferase in a dose-dependent manner. Addition of graded amounts of ACZ885 to a fixed, sub-maximal dose of IL-1β caused a decrease in the expression of luciferase during an incubation period up to 18 hours. At the end of the incubation period, the amount of luciferase was quantified based on its enzymatic activity in the cell lysate. Luciferase catalysed the conversion of the substrate luciferin to oxyluciferin, a chemiluminescent product. The resultant glow-type chemiluminescence was then determined with an appropriate luminometer.

The biological potency of an ACZ885 test sample was determined by comparing its ability to inhibit the IL-1β-dependent induction of luciferase activity to that of an ACZ885 reference standard. The samples and standard were normalized on the basis of protein content. Relative potency was then calculated using a parallel line assay according to the European Pharmacopoeia. The final result was expressed as relative potency (in percent) of a sample compared to the reference standard.

Reagents and buffers:
- Basic medium for cell culture MEM + Earle's + L-Glutamine;
- Fetal calf serum (FCS) heat inactivated, mycoplasma screened;
- Geneticin;
- Cell dissociation buffer enzyme-free, PBS-based;
- Basic medium for assay OptiMEM-I + GlutaMAX-I;
- Luciferase substrate for glow-type chemiluminescence;
- Recombinant Interleukin -1 beta (IL-1β).

Assay procedure steps:
(1) Various concentrations of the reference standard and test samples were prepared by performing several 1:2 dilutions from a starting solution of 400 ng/ml ACZ885;
(2) 2 x 10⁴ cells resuspended in assay media were added to each well of a 96-well microtiter plate;
(3) The assay was started by the addition of an IL-1 beta solution. Incubation in a humidified CO₂ incubator was for up to 18 h;
(4) After the incubation, luciferase substrate solution was added to all wells. The plate was further incubated in the dark for 10 minutes and the luminescence of each well was determined by an appropriate microtiter plate luminescence reader;
(5) The unweighted mean relative potency of a sample was calculated using the parallel line evaluation according to EP from at least two independent experiments.

**Table 8 Analytical results for the technical stability:**

| **Code** | | **Reconstitution time** | **Opalescence** | **pH** | **Assay by UV [mg/ml]** | **Iso-quant iso-asp/ protein [%]** | **Molecular weight by LLS [kDa]** | **Sum of Aggregates by SEC [%]** | **Assay by SEC [mg/ml]** | **Bioassay [%]** |
|---|---|---|---|---|---|---|---|---|---|---|
| F1 | 5°C | 4min | none | 6.2 | 171.8 | 3.8 | 149.2 | 1.0 | 164.8 | 103 |
| | (I) | 45sec | | | | | | | | |
| | 5°C | 4min | none | 6.2 | 172.5 | 3.9 | 149.4 | 1.0 | 167.7 | |
| | (II) | 30sec | | | | | | | | |
| | 25°C | 4min | none | 6.2 | 171.9 | 5.6 | 152.7 | 2.7 | 163.6 | 99 |
| | (I) | 45sec | | | | | | | | |
| | 25°C | 4min | none | 6.2 | 168.4 | 6.3 | 153.3 | 2.7 | 160.0 | |
| | (II) | 45sec | | | | | | | | |
| | 40°C | 4min | none | 6.2 | 172.5 | **9.5** | **161.6** | **7.6** | 152.7 | 93 |
| | (I) | 30sec | | | | | | | | |
| | 40°C | >12min | none | 6.2 | 166.2 | **10.3** | **162.9** | **7.9** | 153.0 | |
| | (II) * | | | | | | | | | |
| F3 | 5°C | 4m in | none | 6.2 | 167.4 | 3.4 | 149.6 | 0.8 | 163.1 | 101 |
| | (I) | 00sec | | | | | | | | |
| | 5°C | 4min | none | 6.2 | 162.8 | 3.1 | 149.1 | 0.8 | 163.0 | |
| | (II) | 00sec | | | | | | | | |
| | 25°C | 4min | none | 6.2 | 159.4 | 4.9 | 151.4 | 1.7 | 158.2 | 91 |
| | (I) | 30sec | | | | | | | | |
| | 25°C | 4min | none | 6.2 | 155.1 | 4.7 | 152.3 | 1.6 | 161.7 | |
| | (II) | 30sec | | | | | | | | |
| | 40°C | 4min | none | 6.2 | 164.4 | 6.6 | 156.6 | 4.5 | 154.9 | 95 |
| | (I) * | 45sec | | | | | | | | |
| | 40°C | 5min | none | 6.2 | 166.01 | 6.1 | 155.7 | 4.5 | 156.8 | |
| | (II) * | 45sec | | | | | | | | |

| | | | | | | | | | | |
|---|---|---|---|---|---|---|---|---|---|---|
| * pieces of the cake stick to the bottom. | | | | | | | | | | |

Formulation 3 showed the lowest amount of aggregation. Formulations 1 and 3 showed a biological activity upon storage of about 90-105 % of the original activity.

### Administration of the Formulation

The appropriate dosage (i.e. therapeutically effective amount) of ACZ885 depends, for example, on the condition to be treated, the severity and course of the condition, whether ACZ885 is administered for preventive or therapeutic purposes, previous therapy, the patient's clinical history and response to ACZ885, and the discretion of the attending physician.
**ACZ885 Heavy chain variable region Seq. Id. No. 1** 
ATGGAGTTTGGGCTGAGCTGGGTTTTCCTCGTTGCTCTTTTAAGAGGTGTCCAGTG
TCAG-19 M E F G L S W V F L V A L L R G V Q C Q-1
 
GTGCAGCTGGTGGAGTCTGGGGGAGGCGTGGTCCAGCCTGGGAGGTCCCTGAGAC
TCTCC V Q L V E S G G G V V Q P G R S L R L S-21
 
TGTGCAGCGTCTGGATTCACCTTCAGTGTTTATGGCATGAACTGGGTCCGCCAGGC
TCCA C A A S G F T F S V Y G M X W V R Q A P-41
 
GGCAAGGGGCTGGAGTGGGTGGCAATTATTTGGTATGATGGAGATAATCAATACT
ATGCA G K G L E W V A 1 1 W Y D G D N Q Y Y A-61
 
GACTCCGTGAAGGGCCGATTCACCATCTCCAGAGACAATTCCAAGAACACGCTGT
ATCTG D S V K G R F T 1 S R D N S K N T L Y L-81
 
CAAATGAACGGCCTGAGAGCCGAGGACACGGCTGTGTATTATTGTGCGAGAGATC
TTAGG Q M N G L R A E D T A V Y Y C A R D L R-101
 
ACTGGGCCTTTTGACTACTGGGGCCAGGGAACCCTGGTCACCGTCTCCTC T G P F
D Y W G Q G T L V T V S S-118
**ACZ885 Light chain variable region Seq. Id. No. 2**

ATGTTGCCATCACAA.CTCATTGGGTTTCTGCTGCTCTGGGTTCCAGCCTCCAGGGG
TGAA-19M L P S Q L I G F L L L W V P A S R G E-1

ATTGTGCTGACTCAGTCTCCAGACTTTCAGTCTGTGACTCCAAAGGAGAAAGTCAC
CATC I V L T Q S P D F Q S V T P K E K V T 1-21

ACCTGCCGGGCCAGTCAGAGCATTGGTAGTAGCTTACACTGGTACCAGCAGAAAC
CAGAT T C R A S Q S 1 G S S L H W Y Q Q K P D-41

CAGTCTCCAAAGCTCCTCATCAAGTATGCTTCCCAGTCCTTCTCAGGGGTCCCCTC
GAGG Q S P K L L I K Y A S Q S P S G V P S R-61

TTCAGTGGCAGTGGATCTGGGACAGATTTCACCCTCACCATCAATAGCCTGGAAG
CTGΛΛ F S G S G S G T D F T L T I N S L E Λ E-81

GATGCTGCAGCGTATTACTGTCATCAGAGTAGTAGTTTACCATTCACTTTCGGCCC
TGGG D A A A Y Y C H Q 5 S S L P F T F G P G-101 ACCAAAGMGATATCAAA-107 T
K V D I K

## Claims

1. A liquid pharmaceutical formulation comprising 10 to 150 mg/mL anti-IL-1β antibody ACZ885, 270 mM sucrose, 20 to 30 mM histidine, and 0.06% polysorbate 80, wherein the pH of the formulation is 6.5.

2. The formulation of claim 1, comprising 30 mM histidine.

3. The formulation of claim 1 or claim 2, comprising 10 mg/mL anti-IL-1β antibody ACZ885.

4. The formulation of claim 1 or claim 2, comprising 150 mg/mL anti-IL-1β antibody ACZ885.

5. A lyophilisate which in reconstituted form yields a reconstituted formulation comprising 10 to 150 mg/mL anti-IL-1β antibody ACZ885, 270 mM sucrose, 20 to 30 mM histidine, and 0.06% polysorbate 80, wherein the pH of the formulation is 6.5.

6. The lyophilisate of claim 5, wherein the reconstituted formulation comprises 30 mM histidine.

7. The lyophilisate of claim 5 or claim 6, wherein the reconstituted formulation comprises 10 mg/mL anti-IL-1β antibody ACZ885.

8. The lyophilisate of claim 5 or claim 6, wherein the reconstituted formulation comprises 150 mg/mL anti-IL-1β antibody ACZ885.

9. A method for preparing a formulation, comprising reconstituting the lyophilisate of any one of claims 5 to 8 with a reconstitution medium.

10. The method of claim 9, wherein the reconstitution medium is sterile water.

11. The method of claim 9, wherein the reconstitution medium is bacteriostatic water for injection (BWFI).

12. A liquid pharmaceutical formulation which is reconstituted from the lyophilisate of any one of claims 5 to 8.

13. The formulation of claim 12, wherein the lyophilisate is reconstituted with sterile water.

14. The formulation of claim 12, wherein the lyophilisate is reconstituted with bacteriostatic water for injection (BWFI).

## Patentansprüche

1. Pharmazeutische Flüssigformulierung, umfassend 10 bis 150 mg/ml Anti-IL-1β-Antikörper ACZ885, 270 mM Saccharose, 20 bis 30 mM Histidin und 0,06 % Polysorbat 80, wobei die Formulierung einen pH-Wert von 6,5 aufweist.

2. Formulierung nach Anspruch 1, umfassend 30 mM Histidin.

3. Formulierung nach Anspruch 1 oder Anspruch 2, umfassend 10 mg/ml Anti-IL-1β-Antikörper ACZ885.

4. Formulierung nach Anspruch 1 oder Anspruch 2, umfassend 150 mg/ml Anti-IL-1β-Antikörper ACZ885.

5. Lyophilisat, das in rekonstituierter Form eine rekonstituierte Formulierung ergibt, die 10 bis 150 mg/ml Anti-IL-1β-Antikörper ACZ885, 270 mM Saccharose, 20 bis 30 mM Histidin und 0,06 % Polysorbat 80 umfasst, wobei die Formulierung einen pH-Wert von 6,5 aufweist.

6. Lyophilisat nach Anspruch 5, wobei die rekonstituierte Formulierung 30 mM Histidin umfasst.

7. Lyophilisat nach Anspruch 5 oder Anspruch 6, wobei die rekonstituierte Formulierung 10 mg/ml Anti-IL-1β-Antikörper ACZ885 umfasst.

8. Lyophilisat nach Anspruch 5 oder Anspruch 6, wobei die rekonstituierte Formulierung 150 mg/ml Anti-IL-1β-Antikörper ACZ885 umfasst.

9. Verfahren zur Herstellung einer Formulierung, umfassend Rekonstituieren des Lyophilisats nach einem der Ansprüche 5 bis 8 mit einem Rekonstituierungsmedium.

10. Verfahren nach Anspruch 9, wobei es sich bei dem Rekonstituierungsmedium um steriles Wasser handelt.

11. Verfahren nach Anspruch 9, wobei es sich bei dem Rekonstituierungsmedium um bakteriostatisches Wasser für Injektionszwecke (BWFI) handelt.

12. Pharmazeutische Flüssigformulierung, die aus dem Lyophilisat nach einem der Ansprüche 5 bis 8 rekonstituiert wird.

13. Formulierung nach Anspruch 12, wobei das Lyophilisat mit sterilem Wasser rekonstituiert wird.

14. Formulierung nach Anspruch 12, wobei das Lyophilisat mit bakteriostatischem Wasser für Injektionszwecke (BWFI) rekonstituiert wird.

## Revendications

1. Formulation pharmaceutique liquide comprenant 10 à 150 mg/ml d'anticorps anti-IL-1β ACZ885, 270 mM de saccharose, 20 à 30 mM d'histidine et 0,06 % de polysorbate 80, le pH de la formulation étant de 6,5.

2. Formulation selon la revendication 1, comprenant 30 mM d'histidine.

3. Formulation selon la revendication 1 ou la revendication 2, comprenant 10 mg/ml d'anticorps anti-IL-1β ACZ885.

4. Formulation selon la revendication 1 ou la revendication 2, comprenant 150 mg/ml d'anticorps anti-IL-1β ACZ885.

5. Lyophilisat qui, sous forme reconstituée, donne une formulation reconstituée comprenant 10 à 150 mg/ml d'anticorps anti-IL-1β ACZ885, 270 mM de saccharose, 20 à 30 mM d'histidine et 0,06 % de polysorbate 80, le pH de la formulation étant de 6,5.

6. Lyophilisat selon la revendication 5, la formulation reconstituée comprenant 30 mM d'histidine.

7. Lyophilisat selon la revendication 5 ou la revendication 6, la formulation reconstituée comprenant 10 mg/ml d'anticorps anti-IL-1β ACZ885.

8. Lyophilisat selon la revendication 5 ou la revendication 6, la formulation reconstituée comprenant 150 mg/ml d'anticorps anti-IL-1β ACZ885.

9. Procédé de préparation d'une formulation, comprenant la reconstitution du lyophilisat selon l'une quelconque des revendications 5 à 8 avec un milieu de reconstitution.

10. Procédé selon la revendication 9, le milieu de reconstitution étant de l'eau stérile.

11. Procédé selon la revendication 9, le milieu de reconstitution étant de l'eau bactériostatique pour injection (BWFI).

12. Formulation pharmaceutique liquide qui est reconstituée à partir du lyophilisat selon l'une quelconque des revendications 5 à 8.

13. Formulation selon la revendication 12, le lyophilisat étant reconstitué avec de l'eau stérile.

14. Formulation selon la revendication 12, le lyophilisat étant reconstitué avec de l'eau bactériostatique pour injection (BWFI).
